Europäisches Patentamt

European Patent Office

Office européen des brevets

(19)

(11) **EP 1 338 242 A1**

(12) **EUROPEAN PATENT APPLICATION**

(43) Date of publication:
27.08.2003 Bulletin 2003/35

(51) Int Cl.⁷: **A61B 5/021**, A61B 5/0285,
A61B 5/0225

(21) Application number: 03250917.6

(22) Date of filing: 14.02.2003

(84) Designated Contracting States:
**AT BE BG CH CY CZ DE DK EE ES FI FR GB GR
HU IE IT LI LU MC NL PT SE SI SK TR**
Designated Extension States:
**AL LT LV MK RO**

(30) Priority: **25.02.2002 US 80703**

(71) Applicant: **COLIN CORPORATION
Komaki-shi Aichi 485 (JP)**

(72) Inventors:
• **Eckerle, Josepf Stephen
Redwood City, California 94062 (US)**
• **Marlow, John
Palo Alto, California 94306 (US)**

(74) Representative: **Shackleton, Nicola et al
Page White & Farrer
54 Doughty Street
London WC1N 2LS (GB)**

(54) **Systems and methods for measuring pulse wave velocity and augmentation index**

(57) A noninvasive system and method of measuring vascular pressure waveforms in a living being includes a tonometric sensor device that reduces, or ideally, eliminates, distortion in the vascular pressure waveforms measured. The data from the vascular pressure waveforms are manipulated to determine cardiovascular conditions of a living being based on a comparison of measured augmentation index and/or pulse wave velocity values to typical values for healthy living beings of similar physiological characteristics.

FIG. 7

EP 1 338 242 A1

## Description

BACKGROUND OF THE INVENTION

### 1. Field of Invention

This invention relates to non-invasively measuring a vascular pressure waveform.

### 2. Description of Related Art

The pulse wave velocity, that is, the velocity at which a pressure wave propagates along a blood vessel, such as an artery, varies depending on the physical characteristics and properties of the blood vessel. Such properties may include the stiffness (elasticity) and geometrical dimensions of the vessel. Elastic properties of blood vessels are known to change with aging and with the onset and development of arteriosclerosis.

Determining the pulse wave velocity depends on accurately measuring pressure waveforms at two distinct locations on the body. The wave propagation time is then derived by determining the time it takes the pressure waveform to travel the distance (d) between the two distinct locations. The pulse wave velocity is then determined as the ratio of the distance (d) between the two distinct locations and the propagation time (t), i. e., (d/t).

Because prior noninvasive blood pressure waveform measurement techniques have proven inaccurate, pulse wave velocity is most commonly measured, for example, by inserting catheters into the vascular system at two distinct locations of an artery. However, inserting of catheters into the body invites the danger of bleeding, as well as the possibility of infection. Therefore, using such catheters to measure pulse wave velocity is not a preferred method of screening for arteriosclerosis or other aging of the vascular system in a living being.

Arterial tonometry, for measuring arterial pressure waveforms, has been used to determine pulse wave velocity indirectly, by noninvasively measuring radial artery blood pressure, at the pulse at one's wrist. This method of arterial tonometry compresses the radial artery between a superficial (external) sensor and the underlying radius bone. A miniature pressure transducer within the sensor then detects the arterial pressure, which is then mathematically transformed to estimate a central aortic pressure waveform.

The same arterial tonometry system or technique is not feasible, at least with a high degree of reliable accuracy, for measuring a pressure waveform in, for example, a carotid artery in a living being's neck. In particular, the tissues of the neck preclude such a tonometer sensor from adequately compressing the carotid artery to yield an accurate pressure waveform as is possible in the radial artery measurement technique.

A pencil-type tonometer has previously been used to noninvasively measure a carotid artery pressure waveform. Such a pencil-type tonometer is not readily usable since it requires considerable expertise and experience in order to obtain a high-fidelity carotid pressure waveform of reliable accuracy. Moreover, motion artifacts can easily, and detrimentally, affect the fidelity of a pressure waveform in a pencil-type tonometer. In addition, the pencil-type tonometer is susceptible to inadvertent motions of a person or of the pencil-type sensor. Such inadvertent motions often render inaccurate the pressure waveform representations obtained from the pencil-type tonometer.

SUMMARY OF THE INVENTION

This invention provides systems and methods that non-invasively measure the pressure waveform of blood traveling through blood vessels in a living being.

This invention separately provides systems and methods that reduce distortion in a non-invasively measured pressure waveform in a living being.

This invention separately provides systems and methods that determine pulse wave velocity of blood traveling through blood vessels in a living being based upon the non-invasively measured pressure waveform and an electro-cardiogram (ECG) and/or a phono-cardiogram (PCG).

This invention separately provides systems and methods that determine an augmentation index of a living being based upon the non-invasively measured pressure waveform.

This invention separately provides systems and methods that determine a likelihood, or existence, of vascular system obstructions, arteriosclerosis, disease, or other deficiencies based upon a comparison of the pulse wave velocity and/or augmentation index value of a living being derived from the non-invasively measured pressure waveform to a corresponding pulse wave velocity or augmentation index value for a healthy living being of similar physiological characteristics.

In various exemplary embodiments of the systems and methods of the invention, a carotid artery pressure waveform may be noninvasively determined by a tonometric sensing device that encircles a living being's neck, such that sensors are located substantially at or on the area of the neck where the carotid artery is located. As a result, the pressure waveform in the carotid artery is detected. The sensor-detected arterial pressure waveform is then used to make a rapid and accurate determination of the pulse wave velocity and/or of an augmentation index of the vascular system of the living being.

The detected or measured vascular pressure waveform is mathematically manipulated to determine a measured pulse wave velocity and/or a measured augmentation index for a living being. Then the measured augmentation index for the living being is compared to a generally accepted augmentation index value for a healthy living being of similar physiological characteristics.

Alternatively, or in addition to, determining and comparing the measured augmentation index to the generally accepted augmentation index value, the measured pulse wave velocity for the living being may be compared to generally accepted pulse wave velocity values typical for healthy living beings.

When the difference between the measured pulse wave velocity and the corresponding typical pulse wave velocity value, or the difference between the measured augmentation index and the corresponding typical augmentation index value, is outside of a predetermined range, then the living being is identified as having, or being at risk of having, vascular system obstructions, deficiencies and/or disease. The non-invasively determined vascular pressure waveform can therefore be useful information, for example, for diagnosing obstructions, and/or hardening, of the carotid artery. Such obstructions, hardening, disease and/or other deficiencies of the vascular system of a living being are typically related to aging and the onset and development of arteriosclerosis.

Various exemplary embodiments of the systems and methods according to this invention include a device that reliably produces a graphical representation of an arterial pressure waveform by controlling the damping conditions of the sensor applied to the carotid artery. Damping the sensor reduces, or, ideally, eliminates, distortions in the measured pressure waveform, such that the data derived from the measured pressure waveform may be meaningfully compared to generally accepted values, for example, for the pulse wave velocity and/or the augmentation index for a healthy living being. Such a comparison requires that the measured waveform have substantially the same shape as a hypothetical perfectly-sensed pressure waveform from which the generally accepted values would be derived.

Perfect, error-free measurement of the pressure waveform is, of course, a hypothetical ideal. In practice, measurement by an invasive catheter can approach the ideal of error-free measurement.

Mathematically manipulating data from catheter-sensed pressure waveforms of living beings of various physiological characteristics, such as height, weight and age, for example, yields ideal pulse wave velocity and ideal augmentation index values that are generally accepted. Similarly, manipulating the measured arterial pressure waveform of a living being yields a measured pulse wave velocity and a measured augmentation index value for that living being. Any substantial distortion of the shape of a measured arterial pressure waveform from the shape of the hypothetical perfectly-sensed arterial pressure waveform would render comparing the two waveforms unreliable for predicting the existence, or likelihood, of vascular obstructions, disease, or other deficiencies in a living being's vascular system based on values derived from the two differently-shaped waveforms.

Various exemplary embodiments of the vascular tonometry systems and methods according to this invention may be used non-invasively to more easily and reliably determine the vascular pressure waveform than the required severe compression of the radial artery to detect pressure in the previous radial artery method. Further, various exemplary embodiments of the vascular tonometric sensor according to this invention may be applied about a living being's neck to produce a substantially continuous recording of, for example, the carotid artery pressure waveform of that living being without generating the motion artifacts and waveform distortions that occur in pencil-type tonometry systems.

Various exemplary embodiments of the vascular tonometry sensor according to this invention include a substantially C-shaped device that fits around a living being's neck. Thus, aligning the sensors against that living being's neck in the area or location of, for example, the carotid artery, is very easily achieved. As a result, the vascular pressure waveform in the carotid artery is accurately sensed without needing to tediously align and realign the device. This reduces the expertise and experience level required to use such exemplary embodiments of the vascular tonometer sensor according to this invention, while still being able to reliably sense the arterial pressure waveform.

These and other features and advantages of this invention are described in, or are apparent from, the following detailed description of various exemplary embodiments of the systems and methods according to this invention.

BRIEF DESCRIPTION OF THE DRAWINGS

Various exemplary embodiments of the systems and methods of this invention will be described in detail with reference to the following figures, wherein:

Figure 1 illustrates a general representation of an exemplary hypothetical perfectly-sensed carotid artery pressure waveform and a general representation of an exemplary distorted carotid artery pressure waveform;

Figure 2 illustrates an exemplary hypothetical perfectly-sensed carotid artery pressure waveform;

Figure 3 illustrates an exemplary measured carotid artery pressure waveform measured using a sensor with low damping;

Figure 4 illustrates an exemplary measured carotid artery pressure waveform measured using a sensor with medium damping;

Figure 5 illustrates an exemplary measured carotid artery pressure waveform measured using a sensor with high damping;

Figure 6 represents a graph of generally accepted augmentation index values for living beings based on the physiological characteristics of the age of living beings;

Figure 7 illustrates an exemplary embodiment of a tonometric sensor according to this invention;

Figure 8 is a schematic representation of the anatomy of a human neck subject to a sensor head according this invention;

Figure 9 is a schematic representation showing in greater detail the various physiological tissues and structures in a human's neck that the tonometer sensor device according to the invention considers in order to produce a high fidelity blood pressure waveform;

Figure 10 is a block diagram of an exemplary arrangement of the various physiological tissues and structures and the sensor used to simulate a blood pressure waveform measurement according to the systems and methods of this invention;

Figure 11 illustrates a general representation of an electrocardiogram and a phonocardiogram;

Figure 12 illustrates an electrocardiogram and an exemplary shaped carotid artery pressure waveform;

Figure 13 illustrates an electrocardiogram, a phonocardiogram and an exemplary shaped carotid artery pressure waveform; and

Figure 14 illustrates an electrocardiogram, a phonocardiogram, an exemplary shaped carotid artery pressure waveform, and a femoral artery pressure waveform.

## DETAILED DESCRIPTION OF EXEMPLARY EMBODIMENTS

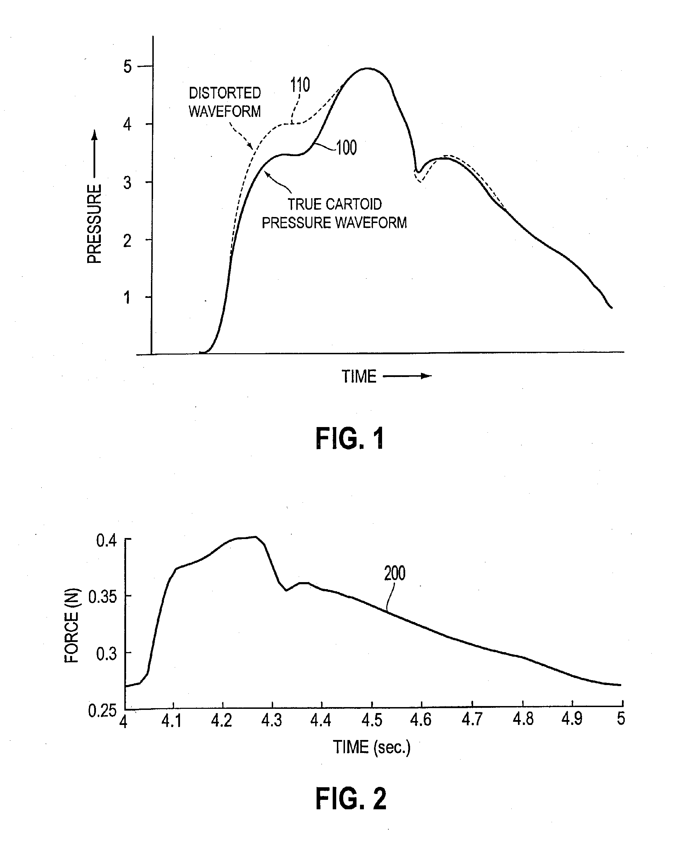

Figure 1 illustrates a general representation of an exemplary hypothetical perfectly-sensed carotid artery pressure waveform 100 of a living being. The hypothetical perfectly-sensed carotid artery pressure waveform 100 is a representation of the shape one could expect a pressure waveform to have, were one able to have a sensor in the aorta to detect the initial and secondary thrusts of blood ejected from the heart or a sensor in the carotid artery, for example, to detect when the initial and secondary thrusts of blood from the heart have reached the carotid artery. The hypothetical perfectly-sensed carotid artery waveform 100 of any being is unique. The hypothetical perfectly-sensed carotid artery pressure waveform is related to several quantifiable factors, such as age, height, weight, and the like, as well as being based on many pertinent non-quantifiable factors, such as mental, emotional and psychological conditions.

Figure 1 further illustrates a general representation of an exemplary distorted carotid artery pressure waveform 110. The exemplary distorted carotid artery pressure waveform 110 represents generally what one might expect a carotid artery pressure waveform to appear as, were the carotid artery pressure actually measured by a generally available pressure sensing device. The shape of the distorted pressure waveform 110 deviates from the shape of the ideal pressure waveform 100 in Figure 1 due to harmonic distortion, bad frequency response or other sensor response characteristics of the sensing device.

The hypothetical perfectly-sensed pressure waveform 100 and the distorted pressure waveform 110 shown in Figure 1 exhibit the same maximum pulse amplitude, or peak, in terms of pressure and time for the second peak of each waveform. However, because of the difference in the shape of the distorted pressure waveform 110 compared to the hypothetical perfectly sensed pressure waveform 100, the first peak of waveforms 100 and 110 differ. As a result, a mathematically derived augmentation index ($A_h$) based on the first and second peaks of the hypothetical perfectly sensed pressure waveform 100 differs from a similarly mathematically derived augmentation index ($A_m$) based on the first and second peaks of the measured pressure waveform 110. Thus, to reliably compare the augmentation index values $A_h$ and $A_m$ derived from the two waveforms 100 and 110, the distortion in the measured pressure waveform 110 needs to be controlled or reduced such that the shape of the distorted waveform 110 more nearly approximates, or, ideally, duplicates, the shape of the ideal waveform 100.

The augmentation index (A) is a mathematically derived value that is determined based upon the first and second peaks, and the maximum and minimum pulse heights of a vascular pressure waveform. Generally, the augmentation index may be determined as:

$$A = (Ph2 - P_{h1}) / (P_{hs} - P_{hd}) \qquad (1)$$

where:

A is the augmentation index corresponding to a hypothetical augmentation index $A_h$, or to a measured augmentation index $A_m$;

$P_{h2}$ is the second peak height in the pressure waveform;

$P_{h1}$ is the first peak height in the pressure waveform;

$P_{hs}$ is the systole pulse height value generally represented as the second peak height in a pressure waveform; and

$P_{hd}$ is the diastole pulse height value represented as the beginning or baseline value in a pressure waveform.

Thus, using the general representations of the exemplary hypothetical perfectly-sensed pressure waveform 100 and the distorted pressure waveform 110 shown in Figure 1, and attributing exemplary values of 1 to 5 along the pressure axis of Figure 1 for each of the pertinent peaks and/or baseline locations of the waveforms, the approximate hypothetical augmentation index $A_h = (5 - 3.4) / (5 - 0) = .32$. However, the approximate measured (distorted) augmentation index $A_m = (5$

- 4) / (5 - 0) = .20 .

The above-derived hypothetical and measured augmentation index values, of course, represent augmentation index values for a living being of the same designated physiological characteristics in terms of height, weight and age, for example. Accordingly, because the approximate hypothetical augmentation index value of .32 is based upon a hypothetical perfectly-sensed pressure waveform, this .32 value could be stored as a generally accepted ideal augmentation index value for one of such designated physiological characteristics. Such generally accepted ideal augmentation index values are then available for future use.

Ideally, the measured (distorted) augmentation index value of .20 would be accurate enough to compare it to the ideal augmentation value of .32 and conclude that the .12 difference between the ideal and measured augmentation index values is either within or beyond an acceptable augmentation index value comparative range of, for example, 0.27 to 0.37 for a living being of similar physical, mental, emotional and psychological characteristics. However, because the measured pressure waveform 110 varies in shape from the hypothetical perfectly-sensed waveform 100, one cannot reasonably rely upon the augmentation index $A_m$ derived from the measured pressure waveform 110 as an accurately reliable representation of that living being's vascular pressure waveform. As a result, one cannot reliably compare the measured augmentation index $A_m$ derived from that distorted waveform 110 to any data derived from catheter measurements--which closely approach the hypothetical perfectly-sensed pressure waveform.

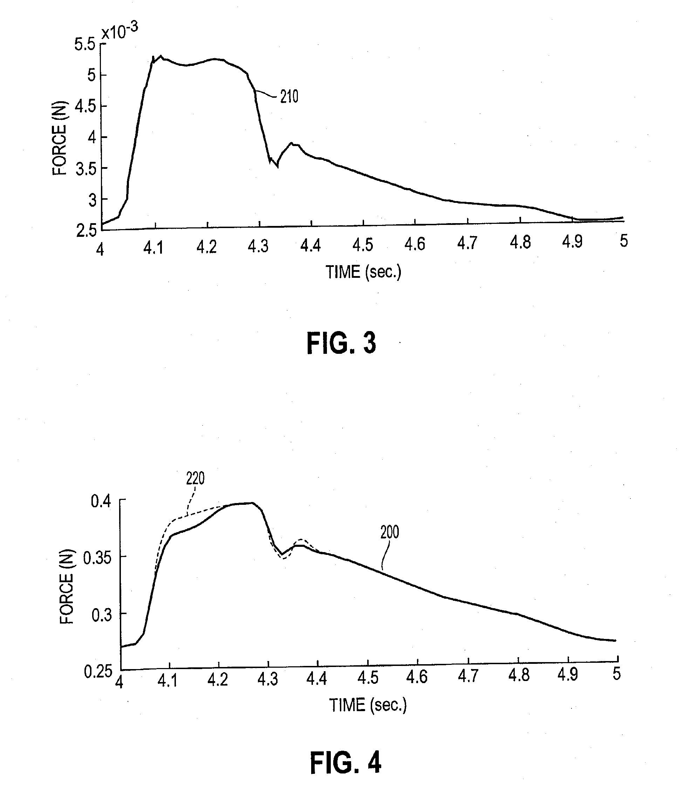

Figure 2 illustrates an exemplary hypothetical perfectly-sensed carotid artery pressure waveform 200 different from that of Figure 1. For comparison purposes relative to Figure 2, Figure 3 illustrates an exemplary measured carotid artery pressure waveform 210 as detected by a tonometer sensor using low damping. The measured pressure waveform 210 of Figure 3 is clearly distorted relative to the pressure waveform of Figure 2.

Similarly, Figure 4 represents an exemplary measured carotid artery pressure waveform 220 resulting from a tonometer sensor using medium damping. Figure 4 also shows a comparison of the measured carotid artery pressure waveform 220 to the same hypothetical perfectly-sensed pressure waveform 200 shown in Figure 2. Though the medium damping results in a measured pressure waveform 220 (dashed line) in Figure 4 that more closely approximates the ideal pressure waveform 200 (solid line) in Figure 4, distortion still occurs. The distortion resulting from the medium damping of the tonometer sensor is evident by the differences between the two waveforms 200 and 220, shown in Figure 4 as superimposed upon one another to emphasize that, though the shapes of the waveforms 200 and 220 are close, the two waveforms 200 and 220 are nonetheless different. As a result of the distortion in the waveform 220, a comparison of an augmentation index based on the hypothetical perfectly-sensed pressure waveform 200 and the measured pressure waveforms 220, for example, would not likely be a reliable indicator of the actual augmentation index values of that living being.

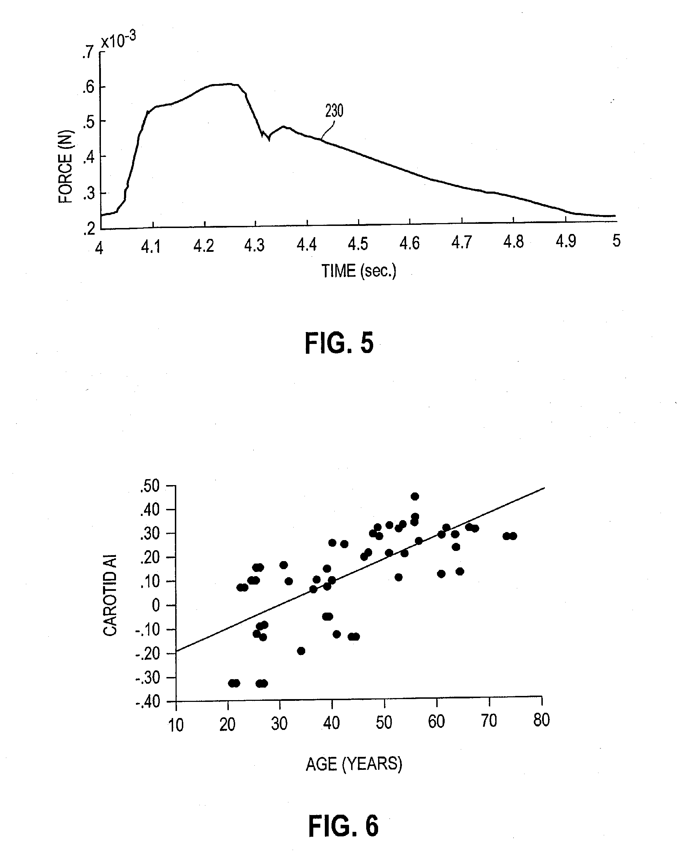

Figure 5 represents a measured artery pressure waveform 230 detected by a tonometer sensor using high damping. Comparing the measured waveform 230 of Figure 5 to the ideally perfectly-sensed pressure waveform 200 of Figure 2, it is evident that as a result of a highly damped sensor, the measured pressure waveform 230 substantially duplicates the shape of the hypothetical perfectly-sensed pressure waveform 200. The only substantial difference between the measured pressure waveform 230 of Figure 5 and the hypothetical perfectly-sensed pressure waveform 200 of Figure 2 is the amplitude (i.e., the vertical scale) of each of the waveforms 230 and 200. Otherwise, the shapes of the two pressure waveforms 230 and 200 are remarkably similar. As a result, the data derived from each of the hypothetical perfectly-sensed pressure waveform 200 of Figure 2 and the measured pressure waveform 230 of Figure 5 will yield accurate and meaningful augmentation indexes that can be reliably compared to one another.

Comparing the augmentation index values derived from each waveform 200 and 230 reveals the amount of difference between these augmentation index values. A physician or a suitably programmed control system may then make a judgment about whether that difference is within or beyond an acceptable range. If the difference between the two augmentation index values is beyond an acceptable range, then that living being is deemed to have, or be at risk of having, possible vascular system obstructions, disease and/or other deficiencies.

Of course, as discussed before with respect to the ideal augmentation index value of .32 derived from the hypothetical perfectly-sensed pressure waveform 100 of Figure 1 for a being of designated physiological characteristics, the ideal augmentation index value based on the hypothetical perfectly-sensed pressure waveform 200 of Figure 2 could also be calculated and stored for future use as a generally accepted augmentation index value for a being having similar physiological characteristics, for example, as the being that generated the waveform 200.

Figure 6 represents a graph of generally accepted augmentation index values for persons of the designated physiological characteristics, which in this case is age. It should be appreciated that similar augmentation index value graphs may be used based on other, or additional, physiological characteristics such as, for example, height, weight, or the like. Likewise, a similar graph or graphs for generally accepted pulse wave velocity values based on one or more physiological characteristics, such as age, height, weight or the like, may also be used.

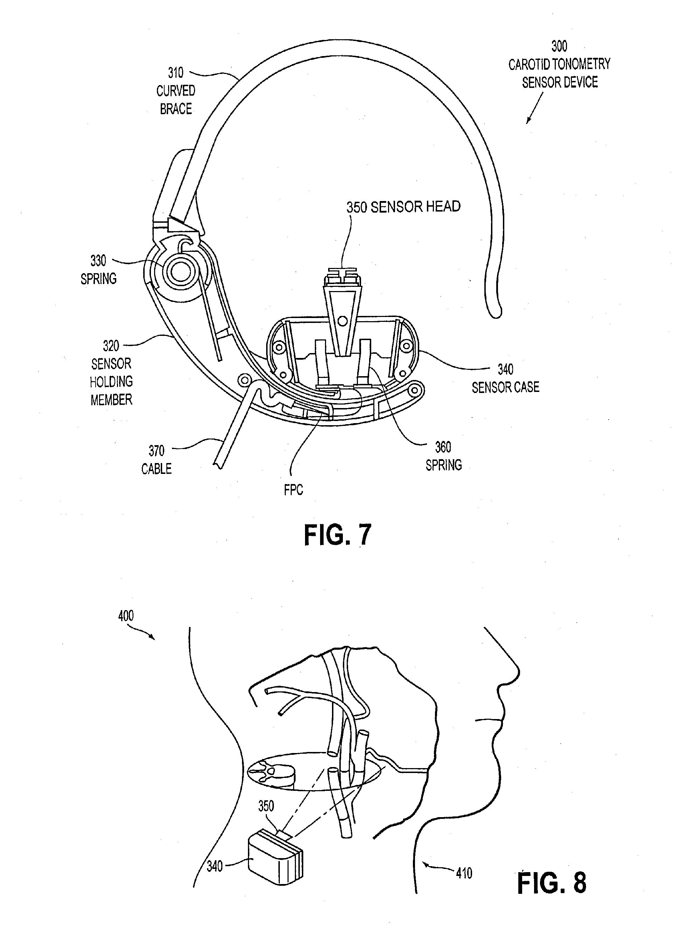

Figure 7 illustrates an exemplary tonometric sensor device 300 according to the systems and methods of this invention. The tonometer sensor device 300 comprises a curved brace 310, a sensor holding member 320, a spring 330, a sensor case 340, a sensor head 350, a spring 360 and a cable 370. The spring 330 joins the curved brace 310 and the sensor holding member 320. The sensor case 340 is fitted to an end of the sensor holding member 320. The sensor case 340 has a spring 360 therein for biasing or controlling movement of the sensor head 350 extending from the sensor case 340 towards the curved brace 310. The angle between the curved brace 310 and sensor holding member 320 can change under the influence of the spring 330 by varying the spring constant of the spring 330 such that the same tonometric sensor device 300 may accommodate human beings, for example, of various sizes or physiological characteristics by fitting the curved brace 310 and sensor holding member 320 with the sensor case 340 and sensor head 350 to the different neck sizes of different human beings.

The angle of the spring 330 between the curved brace 310 and the sensor holding member 320 when no torque acts on the spring 330 is the rest position of the spring 330. The rest position may be varied by positioning the spring 330 at rest such that the angle between the curved brace 310 and the sensor holding member 320 can be changed even in the absence of a change in the spring constant. Of course, it should be appreciated that the positioning of the curved brace 310 relative to the sensor holding member 320 can be made by either a change in the spring constant of spring 330, a change in the resting position of spring 330, or both.

Once fitted around the neck of a human being, the sensor case 340 is positioned such that the sensor head 350 is generally over the location of the carotid artery of the human being. Preferably the sensor head 350 is positioned directly over the carotid artery of the human being. As a result, the pressure of blood traveling through the carotid artery, for example, in the human being may be sensed by the tonometer sensor device 300 and transmitted by a cable 370 to a plotting and/or graphing device. The plotted and/or graphed data can then be mathematically manipulated to derive an augmentation index or a pulse wave velocity value that can be compared to a generally accepted ideal augmentation index value or to a generally accepted pulse wave velocity value previously determined from clinical studies in which pressure waveforms for human beings of the same physiological characteristics are measured by using an invasive catheter.

For the data detected by the exemplary tonometer sensor device 300 to render a blood pressure waveform that can be meaningfully compared to a hypothetical perfectly-sensed blood pressure waveform, however, the tonometer sensor device 300 must account for the various physiological tissues, systems and structures that exist in the general anatomic region of the carotid artery of a living being's neck.

Figure 8 illustrates, in anatomical context, various tissues and structures that a non-invasive sensor, such as, for example, the sensor case 340 and the sensor head 350 need to deal with in order to determine the blood pressure waveform in a blood vessel based upon the blood pressure in, for example, a human being's carotid artery.

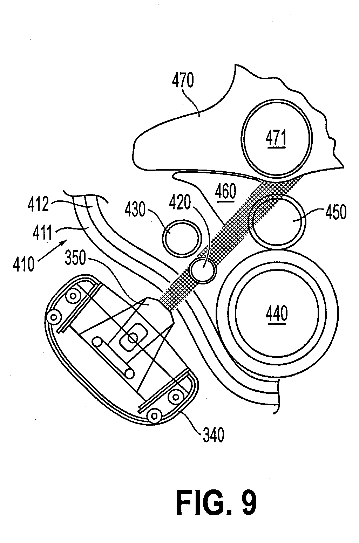

Figure 9 illustrates a more detailed diagram of the anatomical context generally shown in Fig. 8. In Fig. 9, the sensor case 340 positions a single sensor head 350 in contact against the outer surface of skin 411 of the neck 410 of a human 400 and over the general region of the carotid artery 420 that lies beneath the skin 411 and a muscle layer, the platysma 412, just below the skin 411. To either side of the carotid artery 420 are the jugular vein 430 and the trachea 440. Just beyond the trachea 440 is the esophagus 450. Adjacent the esophagus 450 is the rectus capitus anticus major 460, and beyond the esophagus 450 and the rectus capitus anticus major 460 is the vertebrae 470 and the spinal chord 471.

Though other physiological tissues and structures may exist in the same general vicinity of the carotid artery 420, as depicted in Figs. 8 & 9, the identified tissues and structures represent the most significant ones as they are directly in line extending from the sensor head 350, through the carotid artery 420, and ending at the ventral surface of the vertebrae 470. In an approximate idealization of the complex behavior of the anatomy of the neck, we will assume that the behavior will be dominated by the tissues within the cross-hatched portion of Fig. 9.

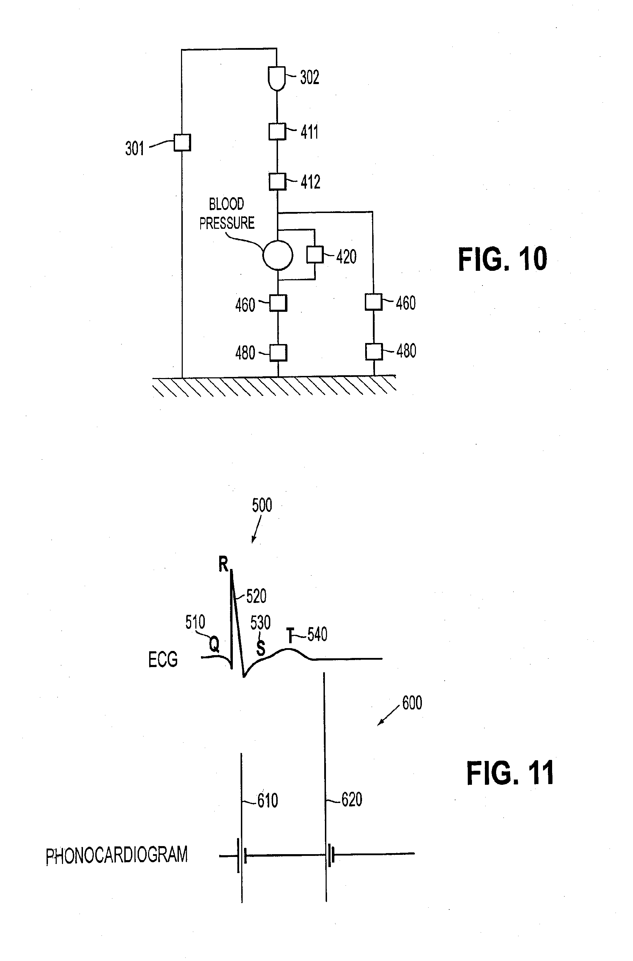

Figure 10 illustrates an exemplary lumped element diagram representing the physiological tissues and structures within the cross-hatched portion, the sensor case 340 and the sensor head 350 shown in Figure 9. The lumped element diagram of Figure 10 thus represents a series of connected lumped elements each representing a distinct portion of one or more of the physical elements shown in Figure 9, configured to simulate the dynamic behavior of blood pressure activities in a carotid artery. The elements configured to simulate the behavior of the sensor and neck tissues include masses, springs, and/or dampers.

A frame 301 of the tonometric sensor device 300 includes those components of the tonometric sensor device 300 that hold the sensor case 340 in place on or about the location of the carotid artery 420. Those components of the frame 301 holding the tonometric sensor device 300 in place include, for example, the curved brace 310, the sensor holding member 320 and the spring 330. Thus, the mass, spring and damper elements of the frame 301 represent the physical characteristics of these components of the tonometric sensor device 300.

As shown in Figure 10, a sensor 302 is mechanically connected between the frame 301 and the skin 411 of the neck 410 of the living being 400. The sensor 302 includes the sensor case 340 and the sensor head 350. The sensor case 340 and the sensor head 350, in particularly, impact directly upon the neck 410 of the living being 400. Thus, the mass, spring and damper elements of the sensor 302 represent the physical characteristics of the sensor case 340 and the sensor head 350.

The links between the frame 301, the sensor 302, and the various tissues of the skin 411, the platysma 412, the carotid artery 420, the rectus capitus anticus major 460 and the longus colli 480 represent energy paths between these elements. More precisely, they represent inter-element forces and the displacements of the inter-element interfaces.

Representing the tonometric sensor device 300 and the various tissues of the skin 411, the platysma 412, the carotid artery 420, the rectus capitus anticus major 460 and the longus colli 480 as lumped elements permits an analysis of the visco-elastic properties of each structure and tissue represented by those blocks. For example, using a Voigt model, the visco-elastic properties of each of these tissues (i.e., the skin 411, the platysma 412, the carotid artery 420, the rectus capitus anticus major 460 and the longus colli 480) is represented by four elements. The four elements are a spring and a damper, connected in parallel, and two masses.

In such a Voigt model, for example, each of the structures 301, 302, 411, 412, 420, 460 and 480 includes a spring element having a spring constant k. The spring constant k is:

$$k = E * w * d / 1 \qquad (2)$$

where:

E is the Young's modulus; and w, d and 1 are the width, depth and length, respectively, of the respective structure or tissue. The skin 411, the platysma 412, the carotid artery 420, the rectus capitus anticus major 460 and the longus colli 480 are each subject to the forces resulting from applying the sensor head 350 to the neck 411 at or on the location of the carotid artery 420 and the pressure of blood in the carotid artery 420, for example.

Various accepted values for E were attributed to the different anatomical tissues and/or structures in order to calculate the spring constant k above for each of these tissues and/or structures. For example, E for bone was deemed to be much greater than $10^7 N/m^2$ ( based upon common knowledge); E for the arterial wall was deemed to be $1.9 \times 10^4$ (Melbin et al., 1988); E for muscle was deemed to be $2.0 \times 10^5$ (Moss & Halpern, 1977); and E for skin was deemed to be $4.5 \times 10^3$ (Potts, et al., 1983). The value of w for each tissue analyzed or used in the simulation was deemed to be the average width of all of the tissues or structures in the cross-hatched region of Figure 9. The depth d of the tonometer sensor head 350 was deemed to be 10 mm, and was used as d for all tissue or structure simulation calculations. The length 1 for each tissue or structure analyzed or used in the simulation was taken from the cross-hatched region of Figure 9 for each respective tissue or structure.

Similarly, each of these structures and/or tissues 301, 302, 411, 412, 420, 460 and 480 includes a damping element or damper. The lumped damping element represents all of the resistive properties of these structures and/or tissues. The damping coefficient of the lumped damping elements is:

$$\beta = \eta * w * d / 1 \qquad (3)$$

where $\eta$ is a viscosity coefficient.

The various values for $\eta$ were similarly assessed according to generally accepted values for each of the various tissues of the neck. For example, $\eta$ for the artery wall was deemed to be 250 N-s/m$^2$ (see Melbin, et al., 1988 for comparison); $\eta$ for muscle was deemed to be 250 (Moss & Halpern, 1977); and $\eta$ for skin was deemed to be 23 (Potts, et al., 1983).

In modeling each of the tissues 411, 412, 420, 460 and 480, in various exemplary embodiments, the spring and damper elements of each tissue are connected in parallel relative to the other elements of these tissues.

In addition to the spring and damping elements, each of the structures 301, 302, 411, 412, 420, 460 and 480 includes a mass element. The lumped mass element represents all of the translational inertial properties of these structures and/or tissues. The mass m of the lumped damping elements is:

$$m = p * w * d * 1 \qquad (4)$$

In this case, the mass m of each tissue was determined using the density of water as $\rho$. For the tissues 411, 412, 420, 460 and 480, the mass m of each tissue was divided into two elements, each representing one-half of the total mass of the corresponding tissue, respectively, to account for the distributed nature of the actual mass of each of these tissues. In modeling these tissues, each of the two mass elements is placed at one end of the parallely-connected spring-damper pair to simulate the effect of the tissue mass on the carotid artery pressure waveform to be generated.

Using the hypothetical perfectly-sensed waveform 200 of Figure 2 as a representative baseline for comparing the simulated carotid artery pressure measurement waveforms, various damping values for the frame portion 301 of the tonometric sensor device 300 were used to produce the simulated waveforms 210, 220 and 230 of Figures 3-5, for example.

Initially, a damping constant of the frame portion 301 of the tonometric sensor device 300 was set to $2.5 * 10^{-3}$ N-s/m. This damping value did not produce a high fidelity waveform, however, as evidenced by the distorted waveform 210 in Figure 3. The deviation or distortion of the waveform 210 shown in Figure 3, produced at a sensor frame damping of $2.5 * 10^{-3}$ N-s/m, is due to resonant oscillation of the sensor head 350, excited by harmonics present in the blood pressure waveform.

Thereafter, a damping constant was set to $2.5*10^{-1}$ N-s/m. This damping value produced the waveform 220 shown in Figure 4. Though not as exaggerated as that of the distorted waveform 210 of Figure 3, distortion in the waveform 220 of Figure 4 was also evident as an obvious deviation from the shape of the hypothetical perfectly-sensed waveform 200 of Figure 2. Accordingly, a higher damping value is desirable to further reduce, or ideally eliminate, distortion in the simulated pressure waveform.

Therefore, damping values were raised to a high damping value of, for example, 250 N-s/m, as shown in Figure 5. This high damping value of 250 N-s/m produced a pressure waveform 230 having a shape sufficiently similar to the hypothetical perfectly-sensed waveform 200 of Figure 2 to permit the data of the measured/sensed simulated waveform to be used as a reliable representation of the dynamic pressure activity in the carotid artery 420 of the living being 400.

As a result of the findings of the simulated pressure waveforms of Figures 3-5 based on the lumped element representation of the tonometric sensor device 300 and various pertinent tissues as shown in Figure 10, a non-invasive tonometric sensor topically applied to the neck of a living being on or at the location of the carotid artery of the living being may reliably represent the arterial pressure waveform of the living being by choosing an appropriately high damping for the tonometer sensor frame. Further by choosing an appropriately high damping, distortions in the pressure waveform may be reduced, or ideally eliminated, and a high fidelity representation of the arterial pressure waveform may be achieved.

Similar to the determination of the augmentation index of a human being as reliably derived from a non-distorted measured pressure waveform for comparison to generally accepted ideal augmentation index values, the pulse wave velocity of a human being may also be reliably derived, at least partly, from a non-distorted measured pressure waveform. However, an additional waveform, different than the measured pressure waveform, is needed to be used together with the pressure waveform in order to determine pulse wave velocity. This differs from the determination of the augmentation index which requires only a single measured pressure waveform for computation of the augmentation index value. The additional waveform required to determine pulse wave velocity of a living being may be one of either an electro-cardiogram (ECG) or a phono-cardiogram (PCG).

Figure 11 illustrates an exemplary representation of the shape of an electrocardiogram 500 and a corresponding phonocardiogram 600. Either of the electrocardiogram 500 or the phonocardiogram 600 may be used with a non-distorted measured pressure waveform, such as the waveform 230 shown in Figure 5, to determine a pulse wave velocity of a living being. The pulse wave velocity of the living being is then compared to a generally accepted pulse wave velocity value for a human being of similar physiological characteristics, to determine whether the being's pulse wave velocity is within an acceptable range in a manner similar to how the augmentation index range was used. If the measured velocity is beyond an acceptable pulse wave velocity range, for one of similar physiological characteristics, then that living being is identified as having, or being at risk of having, possible vascular system obstructions, disease and/or other deficiencies.

The electrocardiogram 500 illustrates the electrical impulses generated by the relaxation and contraction of the muscles of the heart as the heart pumps blood to the body and the lungs. A standard QRST waveform, as shown in the electrocardiogram 500 graphically illustrates the pumping cycle of the heart. The Q portion 510 of the electrocardiogram generally represents the ventricles of the heart at rest and the contraction of the atria of the heart to push the blood from the atria into the ventricles. The R portion 520 of the electrocardiogram 500 represents the beginning of the initial thrust of blood from the ventricles of the heart as the ventricles contract to eject blood from the ventricles to the lungs and body. The end of the R portion 520 of the electrocardiogram 500 represents the end of the initial thrust of blood from the ventricles. The R portion of the QRST waveform is much more prominent because the thrust required to eject blood from the ventricles to the body is much greater than the energy required to thrust blood from the atria to the neighboring ventricles as in the Q portion. After a brief period, represented as the S portion 530 of the electrocardiogram 500, a secondary thrust of blood from the ventricles of the heart occurs as the ventricles complete their contraction and close the aortic and pulmonary valves and deliver blood to the lungs and body. The beginning of the secondary thrust of blood from the heart is represented by the beginning of the T portion 540 of the electrocardiogram 500 as blood is ejected from the ventricles of the heart to the lungs and the body (see, Biology, Peter H. Haven and George B. Johnson, 1986, pages, 982-983). The end of the secondary thrust of blood from the heart is generally represented by the end of the T portion 540 of the electrocardiogram 500. Thus, a complete heart pumping cycle is generally represented by the electrocardiogram 500.

The phonocardiogram 600 is explained in relation to the electrocardiogram 500 discussed above. A phonocardiogram is a graphical representation of the pumping activity of the heart as determined by sound. For example, a stethoscope having a microphone may be used to generate a phonocardiogram of the pumping activities of the heart. The first solid vertical line 610 of the phonocardiogram 600 represents the first heart sound and corresponds with the beginning of the initial thrust of blood from the ventricles of the heart. Thus, vertical line 610 corresponds to the beginning of the R portion 520 of the electrocardiogram 500. The second solid vertical line 620 of the phonocardiogram 600 represents the second heart sound and corresponds with the end of ejection of blood from the left ventricle of the heart. Thus, vertical line 620 corresponds to the closing of the aortic valve.

Generally, pulse wave velocity is a measure of the time t it takes for the pressure in a vessel to travel a distance D from a first location to a second location in a living being. Pulse wave velocity may thus be generally quantified as:

$$V_{PW} = (D_2 - D_1)/(t_2-t_1) \qquad (5)$$

where:

$V_{PW}$ is the pulse wave velocity;
$D_2$ is a second location;
$D_1$ is a first location;
$t_2$ is the time the pressure arrives at the second location; and
$t_1$ is the time pressure leaves the first location.

The electrocardiogram 500, for example, provides a first location $D_1$, which is the heart. The electrocardiogram 500 also provides a first time $t_1$ for example, when the initial thrust of blood pressure has left the heart. The time $t_1$ is at the peak of the R portion 520 of the electrocardiogram 500. However, the electrocardiogram 500 fails to provide information about when that initial thrust of blood has reached a second location $D_2$ a distance $D_2 - D_1$ away from the heart. Nor does the electrocardiogram 500 indicate the time $t_2$ when the initial thrust reaches the second location $D_2$. Thus, to determine pulse wave velocity $V_{PW}$ using Equation (5), a measurement of the same pressure received at another location in the body is required.

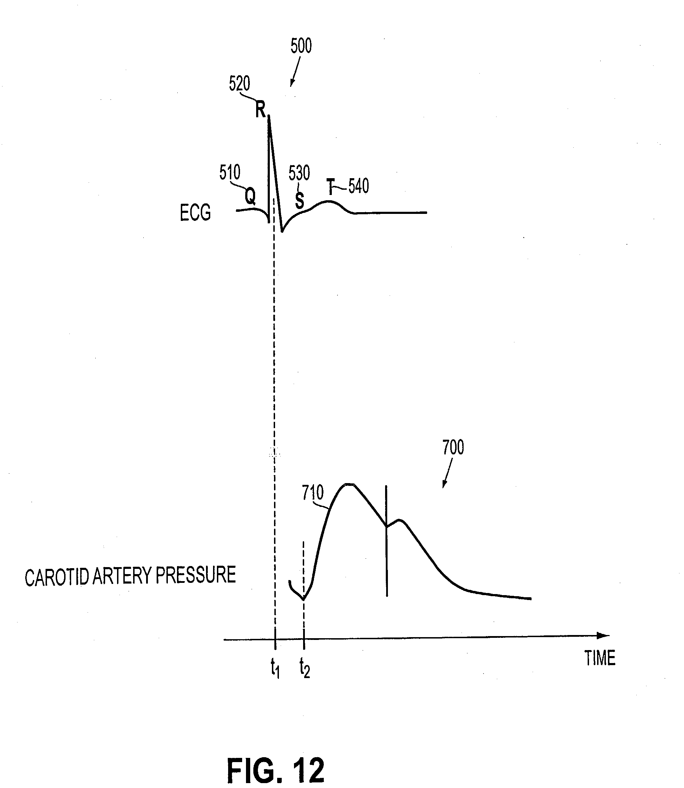

Figure 12 illustrates an electrocardiogram 500 having an exemplary carotid artery pressure waveform 700 plotted on the same graph. The beginning of the upstroke 710 of the carotid artery pressure waveform 700 of Figure 12 indicates the time $t_2$ when the initial thrust of blood that left the heart at the end of the R portion 520 of the electrocardiogram 500 reaches the carotid artery. Thus, the time $t_1$ and time $t_2$ are readily available when the electrocardiogram 500 and the carotid artery pressure waveform 700 are used together.

Further, because the distance from the heart to a carotid artery may be individually measured to determine the distance $D = (D_2 - D_1)$ between a first location $D_1$, such as the heart, and a second location $D_2$, such as the carotid artery, all of the information required to determine pulse wave velocity $V_{PW}$ according to Equation (5) is available. Alternatively, the distance D between two locations on an individual's body may be taken from a table of distance values for individuals based on height, for example. In any case, the distance D from a heart to a second location, such as a carotid artery of an individual where a tonometric sensor may be placed, is readily available.

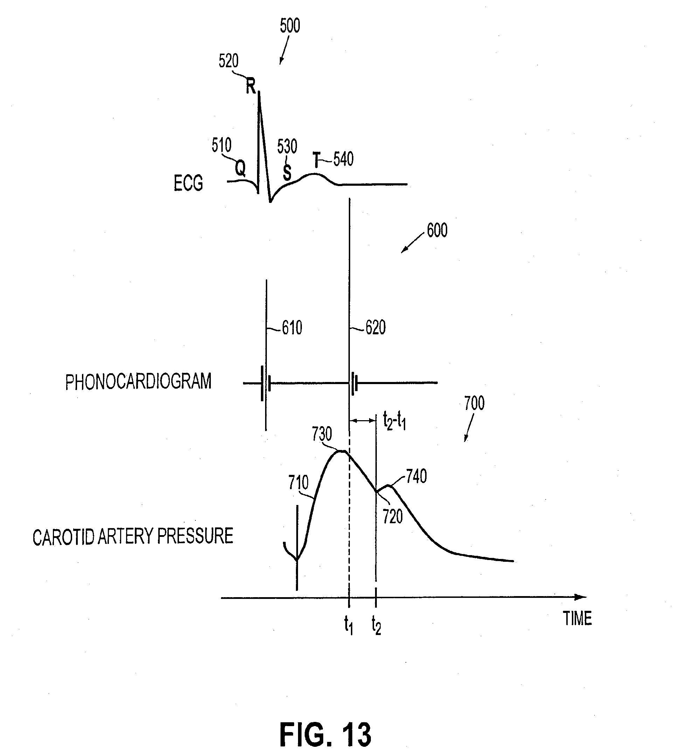

Alternatively, the pulse wave velocity $V_{PW}$ could be determined using a phonocardiogram 600 with, for example, a carotid pressure waveform 700. Figure 13 shows the phonocardiogram 600 and the carotid pressure waveform 700 plotted on the same graph, together with the electrocardiogram 500 for reference.

The second solid line 620 of the phonocardiogram 600 represents the second heart sound, which corresponds to the end of the ejection of blood from the left ventricle of the heart. That is, the second solid line 620 represents when the aortic valve of the heart closes at the end of the ejection of blood from the left ventricle of the heart. The time when the closing of the aortic valve occurs is the time $t_1$.

The carotid pressure waveform 700 shown in Figure 13 has a valley between the first peak 730 and the second peak 740. This valley is known as a dichrotic notch 720. The dichrotic notch 720 of a carotid pressure waveform represents the end of the ejection of blood from the left ventricle of the heart, i.e., the time when the aortic valve closes. Thus, the time when the end of the main thrust of blood from the heart has occurred in the carotid artery is evident by the dichrotic notch 720 of the carotid artery pressure waveform 700 and is thus the time $t_2$. Because of the distance from the heart to the carotid artery of every being, the time $t_2$ evidenced by the dichrotic notch 720 occurs slightly later than the time $t_1$ at the end of the phonocardiogram 600, though both represent closing of the same valve.

As before, the distance D between the heart and the carotid artery of an individual may be either measured directly or retrieved from a table of values for distances from the heart to, for example, the carotid artery of an individual based upon heights.

Having determined all of the necessary information from the phonocardiogram 600 and the carotid pressure waveform of Figure 13, the pulse wave velocity $V_{PW}$ may be determined according to Equation (5 ).

Although the determination of pulse wave velocity $V_{PW}$ has been described above with reference to a carotid artery pressure waveform, such as the carotid pressure waveform 700, it should be appreciated that the pressure waveform of any blood vessel may be used as well to make the same or a similar determination.

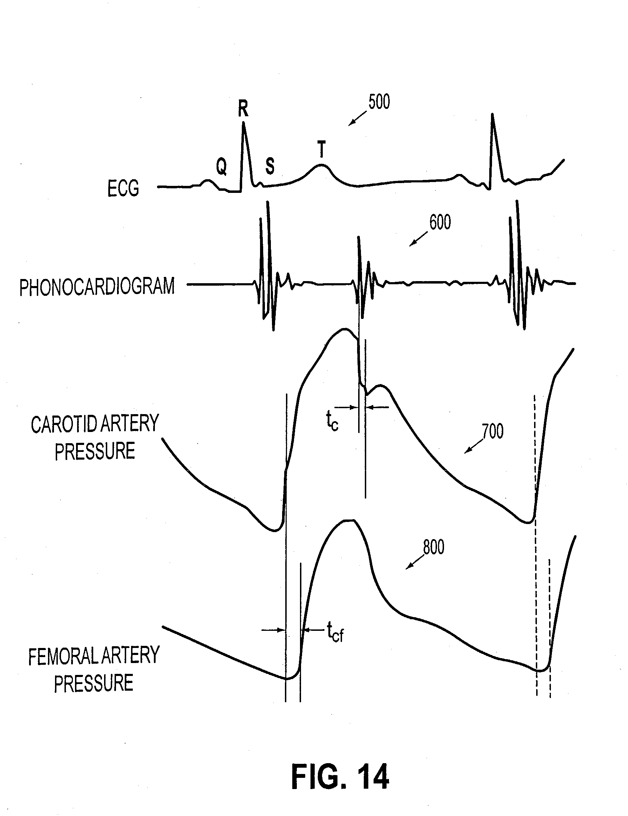

For example, Figure 14 shows a femoral artery pressure waveform 800. Because some pressure waveforms, such as, for example, the femoral artery pressure waveform 800 shown in Figure 14, may not show, for example, the details of the dichrotic notch 720 evident in the carotid pressure waveform 700, it may be desirable to determine the pulse wave velocity $V_{PW}$ according to the electrocardiogram method as discussed above, rather than the phonocardiogram method discussed above.

Alternatively, the pulse wave velocity to the femoral artery may be determined using both carotid artery and femoral artery pressure measurements. Referring to Figure 14, the time difference, $t_c$, is found as described above in reference to Figure 13, $t_c$ being equal to $t_2-t_1$. Next, the time difference $t_{cf}$, between the beginnings of the carotid artery pressure upstroke and the femoral artery pressure upstroke, is found. Then, the pulse wave velocity $V_{PW}$ from the heart to the femoral artery is determined as:

$$V_{pw} = (D2 - D_1)/(t_c + t_{cf}) \qquad (6)$$

where:

$V_{PW}$ is the pulse wave velocity;

$D_2$ is a second location;

$D_1$ is a first location;

$t_c$ is a time difference of the aortic valve closing for the pressure waveforms at the first location, for example the heart, and the second location, for example the carotid artery; and

$t_{cf}$ is a time difference between the beginnings of the pressure upstrokes at the first and second locations.

It should be appreciated that, in addition to controlling damping to reduce, or ideally, eliminate, distortion in the vascular pressure waveforms described above, increasing the spring constant k or changing the resting position of the spring of the sensor frame 301 may be used to have a similar distortion-reducing effect. Similarly, changing the mass m of the frame 301, sensor case 340, or sensor head 350 may also be used to produce a similar distortion-reducing effect in the vascular pressure waveforms.

While this invention has been described in conjunction with the specific embodiments described above, it is evident that many alternatives, combinations, modifications and variations are apparent to those skilled in the art. Accordingly, the preferred embodiments of this invention, as set forth above are intended to be illustrative only, and not limiting. Various changes can be made without departing from the spirit and scope of this invention.

**Claims**

1. A vascular pressure waveform detecting device, comprising:

   at least one sensor usable to sense a vascular pressure waveform;
   a sensor case housing each of the at least one sensor;
   a sensor holding member to which the sensor case is secured; and
   a damping element for the waveform detecting device, the damping element reducing distortion in the vascular pressure waveform sensed by the vascular pressure waveform detecting device.

2. The vascular pressure waveform detecting device according to claim 1, wherein an increase in damping of the damping element reduces distortion in the vascular pressure waveform.

3. The vascular pressure waveform detecting device according to claim 1, further comprising a spring within the sensor holding member, the spring usable to urge the at least one sensor towards a vascular area of a living being, the spring having at least one of a variable spring constant and variable rest position.

4. The vascular pressure waveform detecting device according to claim 1, wherein:

   the at least one sensor has a mass; and
   a change in the mass of the at least one sensor reduces distortion in a vascular pressure waveform sensed by the at least one sensor.

5. The vascular pressure waveform detecting device according to claim 1, wherein:

   the sensor holding member has a mass; and
   a change in the mass of the sensor holding member reduces distortion in a vascular pressure waveform sensed by the at least one sensor.

6. The vascular pressure waveform detecting device according to claim 3, wherein the spring provides reduced distortion in the vascular waveform sensed.

7. A method of determining vascular conditions of a living being, comprising:

   identifying physiologic characteristics of the living being;
   determining an augmentation index value for the living being based on the physiological characteristics identified;

measuring a vascular pressure waveform of the living being using a distortion-reducing vascular pressure waveform detecting device;

determining a measured augmentation index value of the living being from the vascular pressure waveform measured with the distortion-reducing vascular pressure waveform detecting device; and

determining a difference between the measured augmentation index value of the living being and the determined augmentation index value for the living being; and

comparing the difference to an acceptable range of difference for the living being.

8. The method of claim 7, wherein generating the vascular pressure waveform using the distortion reducing vascular pressure waveform detecting device comprises using a distortion reducing vascular waveform detecting device comprising:

at least one sensor usable to sense a vascular pressure waveform;

a sensor case housing each of the at least one sensor;

a sensor holding member to which the sensor case is secured; and

a damping element for the waveform detecting device, the damping element reducing distortion in the vascular pressure waveform sensed by the vascular pressure waveform detecting device.

9. The method of claim 8, further comprising reducing distortion in the vascular pressure waveform by increasing damping provided by the damping element.

10. The method of claim 8, further comprising reducing distortion in the vascular pressure waveform by increasing a spring constant or changing a rest position of a spring that is provided within the sensor holding member and that urges the at least one sensor towards or against a vascular area of a living being.

11. The method of claim 8, further comprising reducing distortion in the vascular pressure waveform by increasing a mass of at least one of the at least one sensor, the sensor case and the sensor holding member.

12. A method of determining vascular conditions of a living being, comprising:

identifying physiologic characteristics of the living being;

determining a pulse wave velocity value for the living being based on the physiological characteristics identified;

generating one of an electrocardiogram and a phonocardiogram of the living being;

generating a waveform based on the generated one of the electrocardiogram and the phonocardiogram;

generating a vascular pressure waveform of the living being using a distortion-reducing vascular pressure waveform detecting device;

comparing the vascular pressure waveform to the generated one of the electrocardiogram waveform and the phonocardiogram waveform to identify a physiological occurrence common to both of the compared waveforms;

determining a first physical location in the living being where the common physiological occurrence shown in one of the two compared waveforms occurs;

determining a second physical location in the living being where the common physiological occurrence shown in the other of the two compared waveforms occurs;

determining a difference in time between the occurrence of the common physiological occurrence in each of the compared waveforms;

determining a pulse wave velocity based on a distance between the first and second locations and the difference in time; and

comparing the pulse wave velocity value to the determined pulse wave velocity for the living being.

13. The method of claim 12, wherein generating the vascular pressure waveform using the distortion reducing vascular pressure waveform detecting device comprises using a distortion reducing vascular pressure waveform detecting device comprising:

at least one sensor usable to sense a vascular pressure waveform;

a sensor case housing each of the at least one sensor;

a sensor holding member to which the sensor case is secured; and

a damping element for the waveform detecting device, the damping element reducing distortion in the vascular pressure waveform sensed by the vascular pressure waveform detecting device.

14. The method of claim 13, further comprising reducing distortion in the vascular pressure waveform by increasing damping provided by the damping element.

**15.** The method of claim 13, further comprising reducing distortion in the vascular pressure waveform by increasing a spring constant or changing a rest position of a spring that is provided within the sensor holding member and that urges the at least one sensor towards or against a vascular area of a living being.

**16.** The method of claim 13, further comprising reducing distortion in the vascular pressure waveform by increasing a mass of at least one of the at least one sensor, the sensor case and the sensor holding member.

**17.** A method of making a vascular waveform detecting device, comprising:

devising simplified mechanical models of the detecting device;
devising simplified mechanical models of physiological tissues corresponding to designated areas of a living being;
combining the simplified mechanical models of the detecting device and the living being to yield a system model of the designated areas of the living being and the detecting device;
using intra-vascular pressure waveform data as an input to drive the system model;
using the system model to simulate the measurement of a vascular pressure waveform of a living being;
comparing the simulated measured waveform to the input waveform to determine waveform distortion;
determining whether the waveform distortion is acceptable for reliable medical use; and
making modifications to the detecting device to render the detecting device more reliable for medical use.

**18.** The method of claim 17 wherein making modifications to the detecting device reduces the distortion in the measured waveform.

**19.** The method according to claim 17, wherein the simplified models of the detecting device and the physiological tissues comprise at least some of springs, masses and dampers.

**20.** The method of claim 17, wherein the detecting device comprises:

at least one sensor usable to sense a vascular pressure waveform;
a sensor case housing each of the at least one sensor; and
a sensor holding member to which the sensor case is secured.

**21.** The method of claim 20, wherein waveform distortion is reduced by increasing damping associated with the sensor holding member.

**22.** The method of claim 20, wherein waveform distortion is reduced by at least one of increasing a spring constant and changing a rest position of a spring associated with the sensor holding member.

**23.** The method of claim 20, wherein waveform distortion is reduced by increasing a mass of at least one of the at least one sensor, the sensor case and the sensor holding member.

DISTORTED
WAVEFORM

110

100

TRUE CARTOID
PRESSURE WAVEFORM

PRESSURE →

5

4

3

2

1

TIME →

**FIG. 1**

FORCE (N)

0.4

0.35

0.3

0.25

200

4    4.1    4.2    4.3    4.4    4.5    4.6    4.7    4.8    4.9    5

TIME (sec.)

**FIG. 2**

**FIG. 3**

**FIG. 4**

**FIG. 5**

**FIG. 6**

300
CAROTID TONOMETRY
SENSOR DEVICE

310
CURVED
BRACE

350 SENSOR HEAD

330
SPRING

320
SENSOR
HOLDING
MEMBER

340
SENSOR CASE

370
CABLE

360
SPRING

FPC

FIG. 7

400

350

340

410

FIG. 8

16

FIG. 9

FIG. 10

FIG. 11

**FIG. 12**

**FIG. 13**

**FIG. 14**

**European Patent**
**Office**

**PARTIAL EUROPEAN SEARCH REPORT**  Application Number

which under Rule 45 of the European Patent Convention EP 03 25 0917
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 561 447 A (KAWAMURA NORIO ET AL) 31 December 1985 (1985-12-31) * column 12, line 14 - column 16, line 21 * | 1-6 | A61B5/021 A61B5/0285 A61B5/0225 |
| Y | * figure 12 * | 17-23 | |
| X | US 6 132 382 A (THEDE ROGER C ET AL) 17 October 2000 (2000-10-17) * column 5, line 18 - column 6, line 46; figure 3B * | 1,2,4,5 | |
| Y | WO 00 72750 A (MASSACHUSETTS INST TECHNOLOGY) 7 December 2000 (2000-12-07) * page 4, line 16 - page 5, line 11 * * page 8 * * abstract * | 17-23 | |
| Y | US 4 771 792 A (SEALE JOSEPH B) 20 September 1988 (1988-09-20) * column 3, line 19 - line 27 * * column 33, line 37 - line 52 * | 17-23 | TECHNICAL FIELDS SEARCHED (Int.Cl.7) A61B |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

1-6,17-23

Claims searched incompletely :

Claims not searched :

7-16

Reason for the limitation of the search:

Article 52 (4) EPC - Diagnostic method practised on the human or animal body

| Place of search | Date of completion of the search | Examiner |
|---|---|---|
| MUNICH | 3 April 2003 | Rodríguez Cossío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 03 25 0917

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2003

| Patent document cited in search report | | Publication date | Patent family member(s) | | Publication date |
|---|---|---|---|---|---|
| US 4561447 | A | 31-12-1985 | JP | 1031368 B | 26-06-1989 |
| | | | JP | 1547566 C | 28-02-1990 |
| | | | JP | 59131327 A | 28-07-1984 |
| | | | JP | 1031367 B | 26-06-1989 |
| | | | JP | 1547567 C | 28-02-1990 |
| | | | JP | 59131328 A | 28-07-1984 |
| US 6132382 | A | 17-10-2000 | CA | 2347716 A1 | 27-04-2000 |
| | | | CN | 1323178 T | 21-11-2001 |
| | | | WO | 0022983 A1 | 27-04-2000 |
| | | | US | 6245022 B1 | 12-06-2001 |
| WO 0072750 | A | 07-12-2000 | JP | 2003500148 T | 07-01-2003 |
| | | | WO | 0072750 A1 | 07-12-2000 |
| | | | US | 6413223 B1 | 02-07-2002 |
| US 4771792 | A | 20-09-1988 | US | 4646754 A | 03-03-1987 |
| | | | AU | 573080 B2 | 26-05-1988 |
| | | | AU | 5546086 A | 10-09-1986 |
| | | | EP | 0215062 A1 | 25-03-1987 |
| | | | JP | 62502307 T | 10-09-1987 |
| | | | WO | 8604801 A1 | 28-08-1986 |
| | | | US | RE34663 E | 19-07-1994 |

EPO FORM P0459

For more details about this annex : see Official Journal of the European Patent Office, No. 12/82